(19) **Europäisches Patentamt**
**European Patent Office**
**Office européen des brevets**

(11) **EP 1 980 235 A1**

(12) # EUROPEAN PATENT APPLICATION

(43) Date of publication:
**15.10.2008 Bulletin 2008/42**

(51) Int Cl.:
**A61K 8/02** (2006.01)     **A61K 8/85** (2006.01)
**A61Q 19/00** (2006.01)     **A61Q 19/10** (2006.01)

(21) Application number: **08250822.7**

(22) Date of filing: **11.03.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **13.03.2007 JP 2007063733**

(71) Applicant: **Riken**
**Wako-shi, Saitama 351-0198 (JP)**

(72) Inventors:
• **Maeda, Mizuo**
**Wako-shi, Saitama 351-0198 (JP)**

• **Sudesh, Kumar K.**
**11800 Minden, Penang (MY)**
• **Iwata, Tadahisa**
**Wako-shi, Saitama 351-0198 (JP)**

(74) Representative: **Bannerman, David Gardner et al**
**Withers & Rogers LLP**
**Goldings House**
**2 Hays Lane**
**London**
**SE1 2HW (GB)**

(54) **Biodegradable oil absorbing film**

(57)     The present invention provides a biodegradable oil absorbing film containing polyhydroxyalkanoate.

**EP 1 980 235 A1**

**Description**

Technical Field

The present invention relates to a biodegradable oil absorbing film (hereinafter simply referred to as an "oil absorbing film," and more particularly, to a sebum absorbing cosmetic and an oil absorbing cleaning product.

Background Art

Recently, the awareness towards the environmental care is increasing, and thus the applications of biodegradable plastics to various products have been investigated. However, the biodegradable plastics are difficult to fabricate, and thus, few of them are actually commercialized due to the problem of increase in manufacturing costs.

In the cosmetic field, cosmetics for removing excess sebum on the skin, i.e., sebum absorbing cosmetics, are known. The excess sebum on the skin surface is not always desirable. In many cases, the oily skin poses a problem especially in a climate of high temperature and high humidity. For example, pimples appear when pores are clogged with cuticle or sebum due to the activation of sebum secretion. The pimples may become inflamed when bacteria act thereon. In order to remove the excess sebum, many sebum absorbing cosmetics have been developed.

As the lifestyle of many people nowadays, they spend a long time outside the home in many cases. Such a lifestyle produces the necessity of carrying disposable skin care products which are easy to use at anytime and anywhere. Especially, sebum absorbing cosmetics (sebum blotting sheet or sebum blotting film) have been widely used as portable skin care products. This is because the sebum blotting sheet or sebum blotting film is small and lightweight, is easy to keep in a pocket or a handbag, and can be used quickly and easily at anytime and any places.

As a sebum absorbing raw material for the face, linen was used at early stages, but recently, paper is used from the viewpoint of the cost. However, a sebum absorption effect of a paper-based sebum absorbing cosmetic is lower than that of a linen-based sebum absorbingcosmetic. In order to improve the effect, a sebumabsorbing cosmetic in which silk yarn is mixed in paper (plant fiber) has been developed, but a sufficient sebum absorption effect has not been achieved yet (Patent Document 1). Recently, the marketing of a porous thermoplastic film-based sebum absorbing cosmetic has been commenced. Such a sebum absorbing film has higher sebum absorption effect compared with the paper-based sebum absorbing cosmetic, but a sufficient sebum absorption effect has not been achieved yet. Further, such a raw material is not biodegradable or does not have a sufficient biodegradable action. As a biodegradable polyester-based sebum absorbing cosmetic, sebum absorbing cosmetics composed of a mixture of polylactic acid fibers and inorganic powders and/or biodegradable organic powders have been developed. However, with respect to the raw materials of this technique, each material alone does not demonstrate the biodegradable effect and are not completely biodegradable (Patent Document 2). Therefore, sebum absorbing cosmetics composed of biodegradable raw material (s) derived from biomass has been desired.

The polylactic acid is known as a biodegradable material, but the polylactic acid does not decompose in the natural environment. However, because the polylactic acid is hydrolyzed under specific conditions, e.g., in compost (high temperature and high humidity conditions: a temperature of 60°C, a humidity of 70%, for example), the polylactic acid is sometimes referred to as a biodegradable material. However, the polylactic acid does not fall under the category of a biodegradable material which can be decomposed by microorganisms in the natural environment.

Also as home cleaning products such as dishcloths, materials having a high oil absorption property are in demand. It is even more preferable that those home cleaning products be biodegradable.

[Patent Document 1] Japanese Patent Application Laid-open No. 2000-139755
[Patent Document 2] Japanese Patent Application Laid-open No. 2004-248910

Disclosure of the Invention

The present invention has been made in view of the above-mentioned respects, and an object of the present invention is to provide various products composed of a biodegradable raw material(s).

The inventors of the present invention carried out intensive research to solve the above-mentioned problems, and found that polyhydroxyalkanoate, which is a biodegradable polymer, has an outstanding oil absorption property. The inventors have invented a biodegradable oil absorbing film based on this finding.

The oil absorbing film of the present invention is completely biodegradable in various natural environments. It is first disclosed by the present invention that various polyhydroxyalkanoates, which are compatible with living bodies and the natural ecosystem, are used as an oil absorbing film.

More specifically, the present invention is as follows.

[1] A biodegradable oil absorbing film, comprising polyhydroxyalkanoate.

[2] The biodegradable oil absorbing film according to [1], wherein the polyhydroxyalkanoate is one kind or two or more kinds selected from 3-hydroxypropionate, 3-hydroxybutyrate, 4-hydroxybutyrate, 3-hydroxyvalerate, 3-hydroxyhexanoate, 3-hydroxyoctanoate, and 3-hydroxydodecanoate.

[3] The biodegradable oil absorbing film according to [1] or [2], further comprising a petroleum-based plastic.

[4] The biodegradable oil absorbing film according to any one of [1] to [3], wherein the biodegradable oil absorbing film is produced by one of methods selected from a solvent casting method, a melt-crystallization method, a spin casting method, and an electrospinning method.

[5] The biodegradable oil absorbing film according to any one of [1] to [4], wherein the biodegradable oil absorbing film is a cosmetic.

[6] The biodegradable oil absorbing film according to [5], wherein the cosmetic is a skin care cosmetic.

[7] The biodegradable oil absorbing film according to [6], wherein the skin care cosmetic is a sebum absorbing film.

[8] The biodegradable oil absorbing film according to [5], wherein the cosmetic is a skin cleaning cosmetic.

[9] The biodegradable oil absorbing film according to [8], wherein the skin cleaning cosmetic is a cleansing agent.

[10] The biodegradable oil absorbing film according to any one of [1] to [4], wherein the biodegradable oil absorbing film is a cleaning product.

The present invention provides an oil absorbing film which is excellent in oil absorption property and is biodegradable. The biodegradable oil absorbing film can be used as, for example, skin care cosmetics such as a sebum absorbing film, and cleaning products such as dishcloths and cleaning tools for lenses used in precision instruments.

The sebum absorbing film of the present invention is excellent in sebum absorption effect, compatible with a living body (skin), and compatible with the environment. When sebum adsorbs to the sebum absorbing film of the present invention, the transparency of the sebum-adsorbed portion changes. This change rate in transparency shows clearly that the film adsorbs sebum. Therefore, a user can easily confirm the sebum absorption effect. Also in this respect, the sebum absorbing film of the present invention has a desirable characteristic as a commercial product.

All the steps of production, use, and final disposal of the oil absorbing film of the present invention are based on a sustainable bio-based step, which is intrinsically neutral from the viewpoint of a carbon cycle.

Brief Description of the Drawings

Fig. 1 is a graph showing the comparison results of oil absorption ability, oil retention ability, and change rate in transparency between P(3HB-co-3HHx) film, P(3HB-*co*-3HV) film, P(3HB) film, and Product A (Comparative Example).

Fig. 2 is a graph showing the comparison results of oil absorption ability, oil retention ability, and change rate in transparency between P(3HB-*co*-3HHx) film, Product A (Comparative Example), Product B (Comparative Example), and Product C (Comparative Example).

Fig. 3 is a graph showing the comparison results of the thickness between P(3HB-*co*-3HHx) film, Product A (Comparative Example), and Product C (Comparative Example).

Best Mode for carrying out the Invention

(Oil Absorbing Film of the Present Invention)

An oil absorbing film having a 100% biodegradability is developed with the present invention. More specifically, the present invention refers to an oil absorbing film having a 100% biodegradability which contains, as a raw material, polyhydroxyalkanoate (PHA) which is biosynthesized by microorganisms using sugars and vegetable oils as raw materials and which is completely decomposed into water and carbon dioxide by catabolic enzymes secreted from other microorganisms.

The oil absorbing film of the present invention contains PHA. The oil absorbing film of the present invention has an outstanding oil absorption property and is biodegradable (film can be partially biodegradable when a non-biodegradable component other than PHA is mixed). Therefore, the oil absorbing film of the present invention is advantageous in that it is compatible with the ecosystem.

The oil absorbing film of the present invention may be used as various products and a material thereof, requiring the oil absorption property. For example, the oil absorbing film of the present invention may be used for cosmetics (skin care cosmetics such as a sebum absorbing film, skin cleaning cosmetics such as a cleansing agent, makeup cosmetics, etc.), food products (oil absorbing health foods, etc.), daily consumer goods (cleaning products such as dishcloths and cleaning tools for lenses used in precisioninstruments),materials for cleaning the environment (film for absorbing oil flowing in the ocean or floating on the water and the like), and materials for cleansing precision instruments.

(Polyhydroxyalkanoate (PHA) for Use in Oil Absorbing Film of the Present Invention)

The PHA used in the oil absorbing film of the present invention is not particularly limited, but polymers formed of one or two or more kinds of those selected from 3-hydroxypropionate, 3-hydroxybutyrate, 4-hydroxybutyrate, 3-hydroxyvalerate, 3-hydroxyhexanoate, 3-hydroxyoctanoate, and 3-hydroxydodecanoate may be used.

Examples of the polymer include 3-hydroxybutyrate homopolymer, 3-hydroxybutyrate-co-3-hydroxyvalerate copolymer, and 3-hydroxybutyrate-co-3-hydroxyhexanoate copolymer. Note that the monomer ratio of the polymer is not particularly limited.

The PHA for use in the oil absorbing filmof the present invention can be synthesized by a fermentation synthetic method. The fermentation synthetic method refers to a method involving culturing microorganisms having a PHA production ability, and then collecting PHAs accumulated in the bacterial cells.

There is no limitation on microorganisms used in the fermentation synthetic method insofar as the microorganisms each has a PHA production ability. As PHA producing bacteria, 60 or more natural microorganisms are known as polyhydroxybutyrate (PHB) producing bacteria, such as *Ralstonia* species and *Alcaligenes* species (e.g., *Ralstonia eutropha,* now known as *Cupriavidus necator* and *Alcaligenes latus).* In these microorganisms, the PHB is accumulated in the bacterial cells. Moreover, known as bacteria producing a copolymer of hydroxybutyrate and other hydroxyalkanoate (s) are, *Ralstonia eutropha* and *Aeromonas caviae* which produce poly(3-hydroxybutyrate-co-3-hydroxyvalerate) and poly(3-hydroxybutyrate-co-3-hydroxyhexanoate), and *Ralstonia eutropha* and *Delftia acidovorans* which produce poly (3-hydroxybutyrate-co-4-hydroxybutyrate), etc.

In general, in the fermentation synthetic method, the PHAs can be accumulated in the bacterial cells by culturing those microorganisms in a common culture medium containing a carbon source, a nitrogen source, inorganic ions, and, as required, other organic ingredients. By adjusting the composition and concentration of the carbon source given to those microorganisms, polymers in various compositions can be obtained. The PHA can be extracted from the bacterial cells by extraction using an organic solvent such as chloroform, or a method involving decomposing bacterial cell components using an enzyme such as lysozyme, and then filtering PHA granules, etc.

Mentioned as one aspect of the fermentation synthetic method is a method involving culturing microorganisms which are transformed by introducing a recombinant DNA containing a PHA synthetic gene, and extracting the PHA generated in the bacterial cells.

The transformant is cultured in a suitable culture medium, and the PHAs are accumulated in the bacterial cells. Mentioned as a used culture medium is a common culture medium containing a carbon source, a nitrogen source, inorganic ions, and, as required, other organic ingredients. When using *Escherichia coli,* glucose and the like are mentioned as the carbon source and substances derived from natural products such as yeast extract, trypton, etc., are mentioned as the nitrogen source. In addition, inorganic nitrogen compounds such as ammonium salt and the like may be contained. It is preferable to perform the culture for 12 to 48 hours under aerobic conditions while adjusting a culture temperature to 30 to 37°C and a pH during the culture to 6. 0 to 8.0. The PHA can be extracted from the bacterial cells by extraction using an organic solvent such as chloroform, a method involving decomposing bacterial cell components using an enzyme such as lysozyme and then filtering PHA granules, and the like. More specifically, the extraction of PHA can be performed by extracting the PHA using a suitable organic solvent from dried bacterial cells which were separated and collected from a culture solution, and then precipitating the resultant with a precipitant.

The molecular weight of PHA used for the oil absorbing film of the present invention is not limited insofar as the effect of the present invention is not adversely affected. The number average molecular weight is usually 100,000 to 1,500,000, and preferably 400,000 to 1,000,000. When the molecular weight of PHA is in the above range, a film can be readily formed.

The oil absorbing film of the present invention can further contain petroleum-based plastics besides PHA. As the petroleum-based plastics used for the oil absorbing film of the present invention, generally-known polypropylene, aliphatic polyester, aromatic polyester, etc., may be used. The addition amount of the petroleum-based plastic can be suitably determined in accordance with physical properties required for the target molded articles. PHA imparts biodegradability to non-biodegradable petroleum-based plastic molded articles.

As a molding material of the oil absorbing film of the present invention, any suitable ingredients generally used for the target molded articles can be added besides the PHAs. For example, when the target molded article is a film, various additives which are generally used for a film, such as lubricants, UV absorbers, weather resistant agents, antistatic agents, antioxidants, thermostabilizers, nucleating agents, flowability improving agents, colorants, and vitamins can be incorporated as required.

Hereinafter, the oil absorbing film of the present invention will be described in more detail with reference to a sebum absorbing film, but the scope of the present invention is not limited thereto.

(Sebum Absorbing Film)

The sebum absorbing film of the present invention refers to a new type of cosmetic, i.e., a skin care product, in the fact that the film is biodegradable and is aliphatic polyester (polyhydroxyalkanoate) which is biosynthesized from biomass by a microorganism and is a renewable source. The sebum absorbing film of the present invention is suitable for absorbing excess sebum from the skin surface. The present invention has a feature in a selected raw material, and the selected raw material is a biodegradable and biocompatible thermoplastic resin, i.e., polyhydroxyalkanoate (PHA). PHA may be processed into a thin porous film by a conventional simple technology used by the plastics industries.

The sebum absorbing film of the present invention may be produced using the PHA as a raw material by any one of the following four methods,i.e.,asolvent casting method, a melt-crystallization method, a spin casting method, or an electro spinning method. By those methods, pores can be formed on the film surface and the degree of crystallization of the film surface can be minimized.

Specifically, the film can be produced as follows.

A solvent cast film can be obtained by dissolving PHA in a volatile organic solvent, pouring the solution into a petri dish or the like, and evaporating the solvent.

A melt-crystallization film can be produced by melting PHA at a temperature equal to or higher than the melting point, and maintaining the resultant for a given period of time at a predetermined temperature.

A spin cast film can be obtained by dropping PHA, which has been dissolved in an organic solvent, on a spinning base, and, simultaneously with dropping the PHA, evaporating the solvent.

An electro spinning film can be produced by applying a voltage to PHA, which has been dissolved in an organic solvent, thereby emitting a sample from a nozzle tip and producing nano-ordered ultrafine fibers, and laminating the fibers.

It should be noted that the films of the present invention are not limited to films obtained by the above methods, and may be obtained by various film formation methods.

As a molding material of the sebum absorbing film of the present invention, various additives, which are generally used for cosmetics such as humectants (polyethylene glycols, polypropylene glycols, butylene glycols, polyglycerins, sugar alcohols, monosaccharides, polysaccharides, amino polysaccharides (chitin, chitosan, etc.), and vitamins), oil, skin-whitening components, antiinflammatory components, colorants, pigments, surfactants, and disinfectants can be incorporated as required.

As physical properties of the sebum absorbing film of the present invention, when the film is composed of a 3-hydroxybutyrate homopolymer (number average molecular weight: 900,000), the glass transition point (Tg) may be 0 to 10°C and the melting point (Tm) may be 170 to 185°C. When the film is composed of a copolymer of 3-hydroxybutyrate and other PHA (number average molecular weight: 400,000 to 1,000,000), the glass transition point (Tg) may be -10 to 10°C and the melting point (Tm) may be 25 to 170°C. The sebum absorbing film of the present invention has a moderate strength, and, for example, the tensile strength may be 10 to 50 MPa, and the elongation to break may be 5 to 1,000%.

The film can be processed into the sebum absorbing film of the present invention when cut into a suitable dimension (e.g., 3 cm $\times$ 3 cm). The thickness of the sebum absorbing film is 10 to 40 $\mu$m.

The sebum absorbing film of the present invention is a sebum absorbing film used for the purpose of cleaning the skin surface and removing excess sebum. Compared to various sebum absorbing films which are already put on the market, the sebum absorbing film of the present invention is novel and has an outstanding property in terms of the environmental compatibility.

Moreover, the sebum absorbing film of the present invention is soft to touch and has an excellent oil absorption ability and an oil retention ability. The oil absorption ability of the sebum absorbing film of the present invention is usually 0.5 g/g or more, and preferably 0.8 g/g or more. The oil retention ability is usually 70% or more, and preferably 75% or more.

The sebum absorbing film of the present invention usually has a porous structure. The porous structure refers to a structure in which many fine pores are formed on the surface and inside of a material. The material having such a porous structure has a large surface area and an excellent substance-absorption ability. When the sebum absorbing film of the present invention is brought into contact with sebum, the sebum is incorporated in the pores, and thus the sebum is adsorbed into a film.

In a conventional sebum absorbing film composed of a plastic, the plastic itself does not have the oil absorption ability. Therefore, in order to adsorb sebum to the film, an additive for absorbing sebum to a film is generally incorporated. The sebum absorbing film of the present invention does not need to be added with the additive for absorbing sebum because the PHA itself has the oil absorption ability. Therefore, the sebum absorbing film of the present invention has advantages in terms of the manufacturing cost and the quality.

When sebum adsorbs to the sebum absorbing film of the present invention, the transparency of the sebum-adsorbed portion, i.e., the transmittance of light, changes. Thus, based on the difference in the transparency between the sebum-adsorbed portion and a portion to which no sebum adsorbs, an effect of removing the sebum can be confirmed by visual observation. The change rate in transparency of the sebum absorbing film of the present invention is usually 50% or more, and preferably 60% or more.

The initial transparency of a PHA film can be adjusted by adjusting the PHA monomer composition and controlling the degree of crystallization. Further, in the production process of a film, it can be adjusted so as to have the film with a specific transparency. Specifically, the transparency can be adjusted by adjusting the composition ratio, type, and degree of crystallization of a copolymer.

The present invention provides biodegradable sebum absorbing films which contain PHA and each has various sebum absorption properties. The PHA-based sebum absorbing film is the same as a conventional non-biodegradable sebum absorbing film in terms of the appearance and the texture. However, the PHA-based sebum absorbing film is biodegradable, shows outstanding oil absorption property, and shows changes in transparency when in contact with oil. The PHA-based sebum absorbing film, when discarded, is automatically decomposed at a place where microbial activity is observed, such as compost, a landfill of refuse, soil, an underwater environment, a marine environment, mangroves, and a wastewater treatment tank. The PHA-based sebum absorbing film can be decomposed under either aerobic conditions or anaerobic conditions, and can be flushed down a toilet.

Examples

Hereinafter, the present invention will be described in more detail with reference to Examples and Reference Examples, but the scope of the present invention is not limited thereto.

[Example 1] Biosynthesis of Polyhydroxyalkanoate (PHA) and Production of PHA Film

(Used strain)

*Ralstonia eutropha* H16 (a wild strain and a mutant strain)

(Carbon source for bacterial growth and PHA synthesis)

Palm oil product, sodium valerate/ sodium propionate, sodium valerate, or sodium hexanoate

(Culturing method)

Cells were first cultured at 30°C for 24 hours in a nutrient-rich culture medium for the preparation of inoculum. Thereafter, 3% (vol/vol) inoculum was transferred to a mineral culture medium containing the above-mentioned carbon source. The cells were further cultured for 72 hours.

(Extraction and purification)

The biosynthesized PHA was extracted from dry microorganism cells using chloroform, then the bacterial cells were filtered, and the filtrate was put in ethanol for polymer precipitation and purification.

(Evaluation of physical properties of PHA)

The melting point (Tm) and the glass transition temperature (Tg) were measured using Differential Scanning Calorimeter (DSC). The tensile strength and the elongation to break were measured with a tensile tester.
The biosynthesized PHA was determined for the properties, and the results are shown in Table 1.

(Production of PHA film)

A PHA film was produced using the biosynthesized PHA. PHA was dissolved in chloroform at room temperature, poured into a petri dish; and dried in a fume hood for 3 to 4 days, thereby obtaining a solvent cast film.

(Evaluation of physical properties of PHA film)

The PHA film was determined for the properties, and the results are shown in Table 1.

[Table 1] Properties of PHA (co)polymer and PHA film made thereof

| PHA (co) polymer | $T_g$ (°C) | $T_m$ (°C) | $M_n$ (x $10^5$) | Tensile strength (MPa) | Elongation to break (%) |
|---|---|---|---|---|---|
| P(3HB) | 4 | 180 | 9.0 | 43 | 5 |

(continued)

| PHA (co) polymer | $T_g$ (°C) | $T_m$ (°C) | $M_n$ (x $10^5$) | Tensile strength (MPa) | Elongation to break(%) |
|---|---|---|---|---|---|
| P(3HB-*co*-20% 3HV) | -1 | 145 | 7.0 | 20 | 50 |
| P(3HB-*co*-10% 3HHx) | -1 | 127 | 4.6 | 21 | 400 |

Tg: Glass transition temperature, $T_m$: Melting point temperature, $M_n$: Number average molecular weight
3HB: 3-hydroxybutyrate, 3HV: 3-hydroxyvalerate, 3HHx: 3-hydroxyhexanoate

[Example 2] Evaluation of Sebum Absorbing Film

The PHA film produced in Example 1 was cut into 2 cm × 2 cm, thereby obtaining a sebum absorbing film (thickness: about 20 μm).

The obtained sebum absorbing film (Example) and a commercially-available sebum absorbing film (Comparative Example) were evaluated for their performances as a sebum absorbing film. More specifically, the oil absorption ability, the oil retention ability, and the change in transparency of the film were determined and were evaluated by the following procedures.

(Measurement of Oil Absorption Ability)

1. The initial weight of a film was measured.
2. The film was immersed in a mineral oil bath for 1 minute, and then taken out from the bath. Then, the liquid was dripped by gravity.
3. Both surfaces were wiped off with a paper wiper, and then the weight of the film was measured, thereby determining the oil absorption ability. The calculation formula of the oil absorption ability is as follows. The oil absorption ability is represented by the amount of oil (g) with respect to the initial film weight (g).

[Equation 1]

$$\text{Oil absorption ability(g/g)} = \frac{\text{Weight of film after immersed in oil (mg)} - \text{Initial film weight (mg)}}{\text{Initial film weight (mg)}}$$

(Measurement of Oil Retention Ability)

1. A film whose oil absorption ability had been measured was directly placed under a steel board covered with aluminum foil. A 2,000* g weight was placed on the board for one minute. Then, the film was removed, and the weight was measured. The calculation formula of the oil retention ability (%) is as follows.
* A force applied by the 2,000 g weight is equivalent to a force applied when pressed by a person's finger.

[Equation 2]

$$\text{Oil retention ability (\%)} = \left( 1 - \frac{\text{Weight of film after immersed in oil (mg)} - \text{Weight of film after pressurized (mg)}}{\text{Weight of film after immersed in oil (mg)}} \right) \times 100$$

(Measurement of Change in Transparency)

The ability of a film to change its appearance (the ability that the film changes in the appearance from opaque to transparent when oil is retained) was determined using the spectrophotometer.

The absorbance of the film was measured at a wavelength which was most suitable for measuring the absorbance of each film. That is: the absorbance of a PHA film (Example) was measured at a wavelength of 500 nm; the absorbance of a commercially-available sebum absorbing film (polypropylene-based) (Comparative Example), Product A, was measured at a wavelength of 550 nm; the absorbance of a commercially-available sebum absorbing film (polyurethane-based, containing silver) (Comparative Example), Product B, was measured at a wavelength of 600 nm; and the absorbance of a commercially-available sebum absorbing film (Japanese paper) (Comparative Example), Product C, was measured at a wavelength of 400 nm.

1. The film was placed in the spectrophotometer, and the initial OD was measured at a suitable wavelength. The initial OD was recorded.

2. The same operations as in the measurement of the oil absorption ability and the oil retention ability were performed, and then the film was again placed in the spectrophotometer for measuring the OD. The calculation formula of the change in transparency(%) is as follows.

[Equation 3]

$$\text{Change rate in transparency of film(\%)} = \frac{\text{OD value of film after immersed in oil} - \text{Initial OD value}}{\text{Initial OD value}} \times 100$$

The measurement results of the P(3HB-*co*-3HHx) film are shown in Table 2.

The measurement results of the P(3HB-co-3HV) film are shown in Table 3.

The measurement results of the P (3HB) film are shown in Table 4.

The measurement results of Product A (Comparative Example) are shown in Table 5.

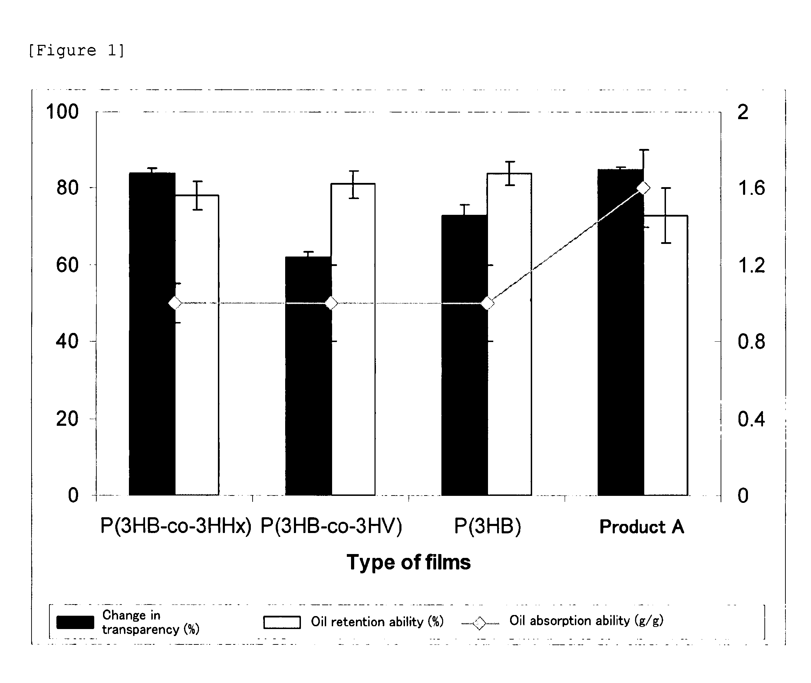

The comparison results (comparison in terms of average) of the oil absorption ability, oil retention ability, and change in transparency between the P(3HB-co-3HHx) film, P(3HB-co-3HV) film, P(3HB) film, and Product A (Comparative Example) are shown in Table 6 and Fig. 1.

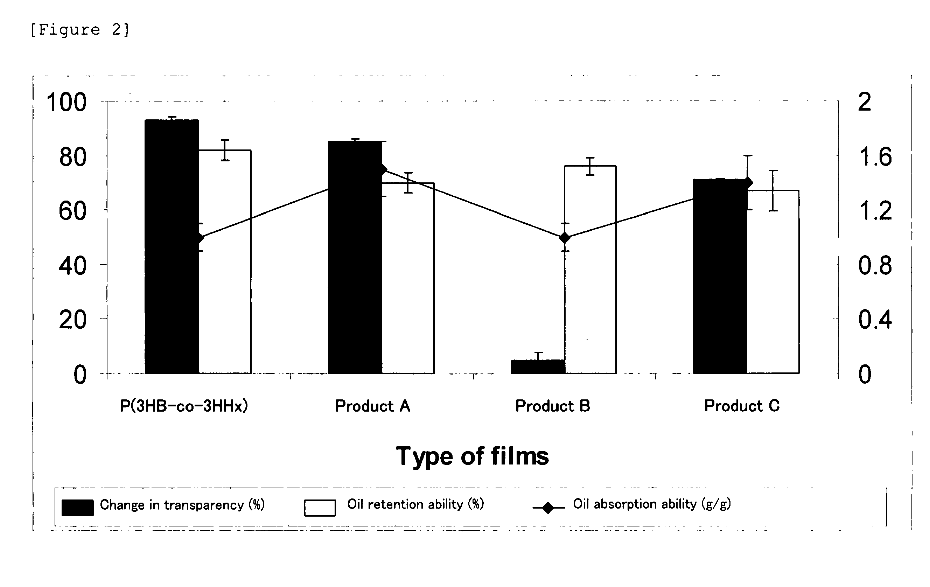

The comparison results (comparison in terms of average) of the oil absorption ability, oil retention ability, and change in transparency between the P(3HB-*co*-3HHx) film, Product A (Comparative Example), Product B (Comparative Example), and Product C (Comparative Example) are shown in Table 7 and Fig. 2.

The results show that the oil absorption ability, oil retention ability, and change in transparency of the sebum absorbing film of the present invention are equal to or higher than those of conventional products.

[Table 2] Properties of P(3HB-co-3HHx) film

| P(3HB-*co*-3HHx) | Film weight (mg) | | | OD | | Change in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 13.8 | 30.9 | 22 | 1.852 | 0.262 | 86 | 1.2 | 71 |
| 2 | 11.1 | 22.7 | 17.6 | 1.762 | 0.272 | 85 | 1 | 78 |
| 3 | 11.3 | 23.4 | 17.8 | 1.704 | 0.247 | 86 | 1.1 | 76 |
| 4 | 15.1 | 29.9 | 23.5 | 1.9 | 0.317 | 83 | 1 | 79 |
| 5 | 13.6 | 28.3 | 22.6 | 1.79 | 0.282 | 84 | 1.1 | 80 |
| 6 | 11.6 | 23.6 | 19 | 1.845 | 0.299 | 84 | 1.1 | 81 |
| 7 | 11.3 | 23.8 | 20 | 1.799 | 0.319 | 82 | 1.1 | 84 |
| Average | | | | | | 84 | 1 | 78 |
| Standard deviation | | | | | | 1.4 | 0.1 | 3.8 |

[Table 3] Properties of P(3HB-co-3Hv) film

| P(3HB-co-3HV) | Film weight (mg) | | | OD | | Change in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 13.8 | 30.3 | 23 | 1.852 | 0.727 | 61 | 1.2 | 76 |
| 2 | 12.9 | 25.7 | 21.1 | 1.884 | 0.684 | 64 | 1 | 82 |
| 3 | 13.5 | 25.3 | 21.8 | 1.721 | 0.662 | 62 | 0.9 | 86 |
| 4 | 13.7 | 32.2 | 24.6 | 1.796 | 0.675 | 62 | 1.4 | 76 |
| 5 | 14 | 27 | 22.5 | 1.753 | 0.685 | 61 | 0.9 | 83 |
| 6 | 12.7 | 27 | 21.6 | 1.646 | 0.654 | 60 | 1.1 | 80 |
| 7 | 15.8 | 29.1 | 24.6 | 1.729 | 0.667 | 61 | 0.8 | 85 |
| Average | | | | | | 62 | 1 | 81 |
| Standard deviation | | | | | | 1.2 | 0.2 | 3.7 |

[Table 4] Properties of P(3HB) film

| P (3HB) | Film weight (mg) | | | OD | | Change in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 12.4 | 27.3 | 22.7 | 1.601 | 0.428 | 73 | 1.2 | 83 |
| 2 | 10.8 | 21.9 | 19.3 | 1.495 | 0.389 | 74 | 1 | 88 |
| 3 | 12.3 | 26.8 | 21.7 | 1.683 | 0.513 | 70 | 1.2 | 81 |
| 4 | 10.7 | 21.2 | 17.6 | 1.367 | 0.286 | 79 | 1 | 83 |
| 5 | 11.2 | 24.3 | 19.1 | 1.599 | 0.415 | 74 | 1.2 | 79 |
| 6 | 14.3 | 25.5 | 21.4 | 1.497 | 0.438 | 71 | 0.8 | 84 |
| 7 | 17.7 | 33.2 | 29.1 | 1.587 | 0.453 | 71 | 0.9 | 88 |
| Average | | | | | | 73 | 1 | 84 |
| Standard deviation | | | | | | 2.8 | 0.2 | 3.1 |

[Table 5] Properties of Product A (Comparative Example)

| Product A | Film weight (mg) | | | OD | | Change in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 8.6 | 27.1 | 15.3 | 4 | 0.596 | 85 | 2.2 | 56 |
| 2 | 8.4 | 22.1 | 16.9 | 4 | 0.592 | 85 | 1.6 | 76 |
| 3 | 9 | 23.8 | 17.8 | 4 | 0.686 | 83 | 1.6 | 75 |
| 4 | 8.5 | 20.8 | 16.2 | 4 | 0.59 | 85 | 1.4 | 78 |

(continued)

| Product A | Film weight (mg) | | | OD | | Change in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 5 | 8.5 | 22.6 | 16.5 | 4 | 0.607 | 85 | 1.7 | 73 |
| 6 | 8.4 | 20.7 | 16.2 | 4 | 0.6 | 85 | 1.5 | 78 |
| 7 | 8 | 20.1 | 15.7 | 4 | 0.622 | 84 | 1.5 | 78 |
| Average | | | | | | 85 | 1.6 | 73 |
| Standard deviation | | | | | | 0.7 | 0.2 | 7.33 |

[Table 6]

Comparison of oil absorption ability, oil retention ability, and change in transparency between P(3HB-co-3HHx) film, P(3HB-co-3HV) film, P(3HB) film, and Product A (Comparative Example)

| | Film | Oil absorption ability (g/g) | Change in transparency (%) | Oil retention ability (%) |
|---|---|---|---|---|
| Example | P(3HB-co-3HHx) | 1±0.1 | 84±1.4 | 78±3.8 |
| Example | P(3HB-co-3HV) | 1±0.2 | 62±1.2 | 81±3.7 |
| Example | P(3HB) | 1±0.2 | 73±2.8 | 84±3.1 |
| Comparative Example | Product A | 1.6±0.2 | 85±0.7 | 73±7.3 |

[Table 7]

Comparison of oil absorption ability, oil retention ability, and change in transparency between P(3HB-co-3HHx) film, Product A (Comparative Example), Product B (Comparative Example), and Product C (Comparative Example)

| | Film | Oil absorption ability (g/g) | Change in transparency (%) | Oil retention ability (%) |
|---|---|---|---|---|
| Example | P(3HB-co-3HHx) | 0.9±0.1 | 93±1 | 82±2 |
| Comparative Example | Product A | 1.5±0.2 | 85±1.0 | 70±4.9 |
| Comparative Example | Product B | 1.0±0.1 | 5±4.8 | 76±4.0 |
| Comparative Example | Product C | 1.4±0.2 | 71±1.5 | 67±3.2 |

[Example 3] Evaluation of Influences on Film Performance by Cleaning treatment

The PHA film produced in Example 1 was immersed in oil, and the first experiment (evaluation of performance) was carried out following the procedure of Example 2. Thereafter, the resulting film was immersed in a commercially-available oil remover (liquid), and the adsorbed oil was removed by stirring overnight. Then, the resulting film was dried, and subjected to the same experiment as that of the first experiment. The number of each film in Tables 8 to 11 designates the number of each of seven different films (i.e., the results shown in Tables 8 to 11 were not obtained by performing the above-mentioned operation 7 times in the same one film).

The measurement results of the P(3HB-co-3HHx) film are shown in Table 8.

The measurement results of the P(3HB-co-3HV) film are shown in Table 9.

The measurement results of the P (3HB) film are shown in Table 10.

The measurement results of Product A (Comparative Example) are shown in Table 11.

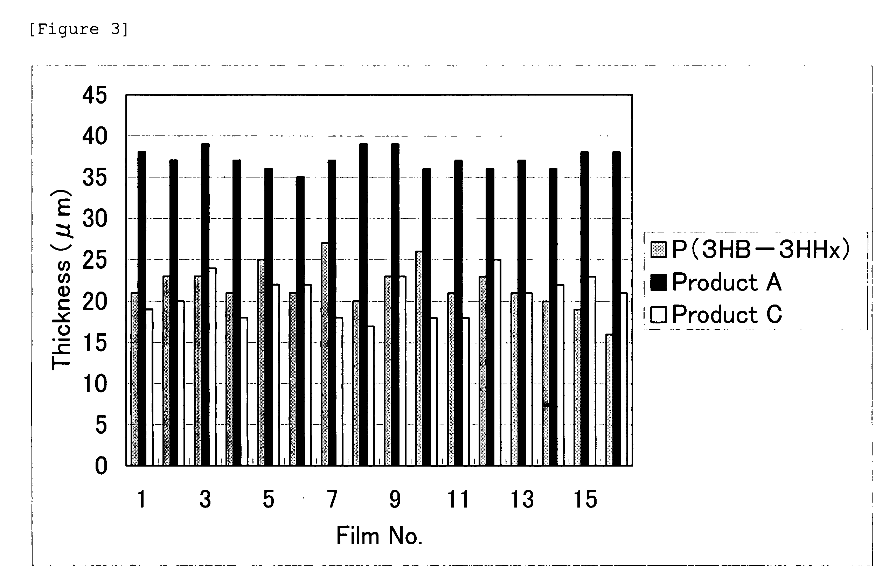

The comparison results (comparison in terms of average) of the oil absorption ability, oil retention ability, and change in transparency between the P(3HB-co-3HHx) film, P(3HB-co-3HV) film, P (3HB) film, and Product A (Comparative Example) are shown in Table 12 and Fig. 3.

[Table 8] Properties of P(3HB-co-3HX) film after washed once

| P(3HB-co-3HHx) | Film weight (mg) | | | OD | | Change rate in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 11.4 | 24.4 | 17.5 | 1.665 | 0.448 | 73 | 1.1 | 72 |
| 2 | 16.2 | 28.8 | 22.4 | 1.563 | 0.555 | 64 | 0.8 | 78 |
| 3 | 11.9 | 23.6 | 17.8 | 1.468 | 0.437 | 70 | 1 | 75 |
| 4 | 15.4 | 25.8 | 21.6 | 1.597 | 0.542 | 66 | 0.7 | 84 |
| 5 | 15.7 | 27.1 | 21.8 | 1.423 | 0.536 | 62 | 0.7 | 80 |
| 6 | 13.3 | 22.9 | 18.9 | 1.517 | 0.468 | 69 | 0.7 | 83 |
| 7 | 12.8 | 26 | 20.1 | 1.621 | 0.464 | 71 | 1 | 77 |
| Average | | | | | | 68 | 0.9 | 78 |
| Standard deviation | | | | | | 3.7 | 0.2 | 4.0 |

[Table 9] Properties of P(3HB-co-3HV) film after washed once

| P(3HB-co-3HV) | Film weight (mg) | | | OD | | Change rate in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 14 | 27.2 | 22.3 | 1.382 | 0.517 | 63 | 0.9 | 82 |
| 2 | 14.7 | 26.5 | 22.1 | 1.446 | 0.663 | 54 | 0.8 | 83 |
| 3 | 14.3 | 27.5 | 23.3 | 1.43 | 0.598 | 58 | 0.9 | 85 |
| 4 | 16.2 | 30.8 | 26.1 | 1.38 | 0.662 | 52 | 0.9 | 85 |
| 5 | 14.4 | 25.7 | 22.5 | 1.38 | 0.648 | 53 | 0.8 | 88 |
| 6 | 14.5 | 25.7 | 20.4 | 1.51 | 0.731 | 52 | 0.8 | 79 |
| 7 | 15.4 | 27.3 | 22 | 1.423 | 0.651 | 54 | 0.8 | 81 |
| Average | | | | | | 55 | 0.8 | 83 |
| Standard deviation | | | | | | 3.7 | 0.1 | 2.8 |

[Table 10] Properties of P(3HB) film after washed once

| P(3HB) | Film weight (mg) | | | OD | | Change rate in transparency (%) | Oil absorption ability (g/g) | Oil retention. ability (%) |
|---|---|---|---|---|---|---|---|---|
| | Initial | After immersed in oil | Oil retention amount | Before retaining oil | After retaining oil | | | |
| 1 | 15 | 27 | 23.7 | 1.299 | 0.537 | 59 | 0.8 | 88 |
| 2 | 12.8 | 20.8 | 19.2 | 1.186 | 0.479 | 60 | 0.6 | 92 |
| 3 | 13.7 | 22.5 | 20.9 | 1.271 | 0.558 | 56 | 0.6 | 93 |
| 4 | 15.9 | 26.3 | 22.4 | 1.215 | 0.567 | 53 | 0.7 | 85 |
| 5 | 18.3 | 30.5 | 27.2 | 1.256 | 0.658 | 48 | 0.7 | 89 |
| 6 | 11.1 | 22.3 | 17 | 1.117 | 0.353 | 68 | 1 | 76 |
| 7 | 11.6 | 24.1 | 19.5 | 1.244 | 0.439 | 65 | 1.1 | 80 |
| Average | | | | | | 58 | 0.8 | 86 |
| Standard deviation | | | | | | 6.3 | 0.2 | 5.8 |

[Table 11] Properties of Product A (Comparative Example) after washed once

| Product A | Film weight (mg) | | | OD | | Change rate in transparency (%) | Oil absorption ability (g/g) | Oil retention ability (%) |
|---|---|---|---|---|---|---|---|---|
| | Initial | After immersed in oil | Oil retention amount | Before retaining oil | After retaining oil | | | |
| 1 | 13.1 | 22.8 | 16.2 | 1.11 | 0.62 | 56 | 0.7 | 71 |
| 2 | 13 | n.d. | 16.3 | 0.97 | 0.593 | 61 | n.d. | n.d. |
| 3 | 13.2 | 20.4 | 10.3 | 1.268 | 0.621 | 49 | 0.5 | 50 |
| 4 | 12.7 | 20.3 | 16.5 | 1.22 | 0.609 | 50 | 0.6 | 81 |
| 5 | 12.3 | 20.5 | 15.9 | 1.288 | 0.606 | 47 | 0.7 | 78 |
| 6 | 14.2 | 21.5 | 17.1 | 1.329 | 0.649 | 49 | 0.5 | 80 |
| 7 | 12.8 | 22.7 | 17.4 | 1.291 | 0.638 | 49 | 0.8 | 77 |
| Average | | | | | | 52 | 0.6 | 73 |
| Standard deviation | | | | | | 4.7 | 0.1 | 10.7 |

[Table 12]

| Comparison of oil absorption ability, oil retention ability, and change in transparency between P(3HB-co-3HHx) film, P(3HB-co-3HV) film, P(3HB) film, and Product A (Comparative Example), which were washed once | | | | |
|---|---|---|---|---|
| | Film | Oil absorption ability (g/g) | Change rate in transparency (%) | Oil retention ability (%) |
| Example | P(3HB-co-3HHx) | 0.9±0.2 | 68±3.7 | 78±4.0 |
| Example | P(3HB-co-3HV) | 0.8±0.1 | 55±3.7 | 83±2.8 |
| Example | P(3HB) | 0.8±0.2 | 58±6.3 | 86±5.8 |
| Comparative Example | Product A | 0.6±0.1 | 52±4.7 | 73±10.7 |

The results show that the oil absorption ability, oil retention ability, and change in transparency of the sebum absorbing film of the present invention are equal to or higher than those of a conventional product.

[Example 5] Evaluation of Thickness of Sebum Absorption Film

16 sebum absorbing films were randomly taken out from the sebum absorbing films produced in Example 1, and the thickness of each film was measured with a thickness meter manufactured by Mitsutoyo Corporation.
With respect to Product A and Product C, the thickness of each of randomly selected 16 films was measured. The results are shown in Table 13 and Fig. 4.

[Table 13]

| Comparison of thickness between P(3HB-co-3HHX) film, Product A, and Product C (Comparative Example) (unit: μm) | | | |
|---|---|---|---|
| Film | Example | Comparative Example | Comparative Example |
| | P(3HB-co-3HHx) | Product A | Product C |
| 1 | 21 | 38 | 19 |
| 2 | 23 | 37 | 20 |
| 3 | 23 | 39 | 24 |
| 4 | 21 | 37 | 18 |
| 5 | 25 | 36 | 22 |
| 6 | 21 | 35 | 22 |
| 7 | 27 | 37 | 18 |
| 8 | 20 | 39 | 17 |
| 9 | 23 | 39 | 23 |
| 10 | 26 | 36 | 18 |
| 11 | 21 | 37 | 18 |
| 12 | 23 | 36 | 25 |
| 13 | 21 | 37 | 21 |
| 14 | 20 | 36 | 22 |
| 15 | 19 | 38 | 23 |
| 16 | 16 | 38 | 21 |

The results show that the sebum absorbing film of the present invention is thin and has a uniform thickness, and has an excellent oil absorption ability.

Industrial Applicability

The present invention can provide an oil absorbing film obtained from aliphatic polyester alone as a raw material having biodegradability which is synthesized from sugars or vegetable oils by microorganisms. For example, the sebum absorbing film of the present invention is effective in that the film production process is simple and the sebum absorbing film can be readily produced, unlike a conventional product which is made of blended materials or a paper made of plant fibers.
Further, even when the sebum absorbing film of the present invention becomes a garbage to be abandoned in the environment, the film is decomposed very promptly by microorganisms in the environment. Therefore, the sebum absorbing film of the present invention can contribute to mitigation of environmental problems. In particular, while polylactic acid is not decomposed in the natural environment, the film of the present invention decomposes within several months in the natural environment, which makes it significantly different from a conventional product. The disposal of the film after use is easy in the respect that there arises no problem when the film is flushed down a toilet, for example.

**Claims**

1. Use of a biodegradable oil absorbing film comprising polyhydroxyalkanoate, for absorbing oil.

2. Use according to claim 1, wherein the polyhydroxyalkanoate is one kind or two or more kinds selected from 3-hydroxypropionate, 3-hydroxybutyrate, 4-hydroxybutyrate, 3-hydroxyvalerate, 3-hydroxyhexanoate, 3-hydroxyoctanoate, and 3-hydroxydodecanoate.

3. Use according to claim 1 or 2, wherein the film further comprises a petroleum-based plastic.

4. Use according to any one of claims 1 to 3, wherein the film is produced by one of methods selected from a solvent casting method, a melt-crystallization method, a spin casting method, and an electrospinning method.

5. Use of a biodegradable oil absorbing film comprising polyhydroxyalkanoate for making up.

6. Use of a biodegradable oil absorbing film comprising polyhydroxyalkanoate for performing skin care.

7. Use of a biodegradable oil absorbing film comprising polyhydroxyalkanoate for absorbing a sebum.

8. Use of a biodegradable oil absorbing film comprising polyhydroxyalkanoate for cleaning skin.

9. Use of a biodegradable oil absorbing film comprising polyhydroxyalkanoate for cleansing skin.

10. Use of a biodegradable oil absorbing film comprising polyhydroxyalkanoate for cleaning.

11. A biodegradable film used for absorbing oil, wherein the film comprises polyhydroxyalkanoate.

12. The biodegradable film according to claim 11, wherein the absorbing oil is absorbing a sebum.

[Figure 1]

EP 1 980 235 A1

[Figure 2]

**Type of films**

| ■ Change in transparency (%) | □ Oil retention ability (%) | ◆ Oil absorption ability (g/g) |

EP 1 980 235 A1

[Figure 3]

**European Patent Office**

**EUROPEAN SEARCH REPORT**

Application Number

EP 08 25 0822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 990 271 A (NODA ISAO [US]) 23 November 1999 (1999-11-23) | 11,12 | INV. A61K8/02 A61K8/85 A61Q19/00 A61Q19/10 |
| A | * claims 1-6; examples 5-7 * * column 3, line 20 - column 7, line 23 * | 1-11 | |
| X | WO 02/077080 A (PROCTER & GAMBLE [US]) 3 October 2002 (2002-10-03) | 6,8-12 | |
| A | * paragraph [0038] - paragraph [0053]; claims 1-10 * * paragraph [0011] - paragraph [0027] * * paragraph [0066] - paragraph [0069] * | 1-11 | |
| X | WO 99/23146 A (MONSANTO CO [US]) 14 May 1999 (1999-05-14) | 11,12 | |
| A | * page 23, line 8 - page 25, line 30; claims * * page 3, line 15 - page 4, line 11 * * page 5, line 21 - page 6, line 29 * | 1-10 | |
| X | US 5 618 855 A (NODA ISAO [US]) 8 April 1997 (1997-04-08) * column 3, line 45 - column 4, line 22; claims; examples 11-13 * * column 11, line 14 - column 16, line 38 * | 6,8-12 | **TECHNICAL FIELDS SEARCHED (IPC)** A61K A61Q |
| A | * column 24, line 35 - column 26, line 62 * | 1-10 | |
| Y | WO 99/29220 A (MINNESOTA MINING & MFG [US]; KONDO KAZUNORI [JP]; ETCHU KUMIKO [JP]) 17 June 1999 (1999-06-17) * page 1, line 4 - line 14; claims; examples * * page 4, line 15 - page 22, line 9 * | 1-5,7 | |
| Y | CN 1 313 348 A (LI FASHENG [CN]) 19 September 2001 (2001-09-19) * abstract * | 1-5,7 | |

-/--

The present search report has been drawn up for all claims

| Place of search | Date of completion of the search | Examiner |
|---|---|---|
| Munich | 28 July 2008 | Loloiu, Teodora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 08 25 0822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 1 405 220 A (LI FASHENG [CN]) 26 March 2003 (2003-03-26) * abstract * ----- | 1-5,7 | |
| A | REDDY C.S.K., GHAI R., RASHMI, KALIA V.C.: "Polyhydroxyalkanoates: an overview" BIORESOURCE TECHNOLOGY, ELSEVIER SCIENCE, [Online] vol. 87, no. 2, April 2003 (2003-04), pages 137-146, XP002489595 Retrieved from the Internet: URL:http://dx.doi.org/10.1016/S0960-8524(0 2)00212-2> [retrieved on 2008-07-23] * the whole document * ----- | 1-12 | |
| | | | TECHNICAL FIELDS SEARCHED (IPC) |

The present search report has been drawn up for all claims

| Place of search | Date of completion of the search | Examiner |
|---|---|---|
| Munich | 28 July 2008 | Loloiu, Teodora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 25 0822

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2008

| Patent document cited in search report | | Publication date | Patent family member(s) | | Publication date |
|---|---|---|---|---|---|
| US 5990271 | A | 23-11-1999 | AT | 193310 T | 15-06-2000 |
| | | | AU | 685224 B2 | 15-01-1998 |
| | | | AU | 1568095 A | 15-08-1995 |
| | | | BR | 9506590 A | 16-09-1997 |
| | | | CA | 2181796 A1 | 03-08-1995 |
| | | | CN | 1139938 A | 08-01-1997 |
| | | | CZ | 9602210 A3 | 15-01-1997 |
| | | | DE | 69517157 D1 | 29-06-2000 |
| | | | DE | 69517157 T2 | 25-01-2001 |
| | | | DK | 741757 T3 | 07-08-2000 |
| | | | EP | 0741757 A1 | 13-11-1996 |
| | | | ES | 2145903 T3 | 16-07-2000 |
| | | | FI | 962998 A | 26-07-1996 |
| | | | GR | 3033467 T3 | 29-09-2000 |
| | | | HK | 1013090 A1 | 30-03-2001 |
| | | | HU | 75181 A2 | 28-04-1997 |
| | | | JP | 3501809 B2 | 02-03-2004 |
| | | | JP | 9508426 T | 26-08-1997 |
| | | | NZ | 279143 A | 27-05-1998 |
| | | | PL | 315684 A1 | 25-11-1996 |
| | | | PT | 741757 T | 31-10-2000 |
| | | | RU | 2132342 C1 | 27-06-1999 |
| | | | SG | 49096 A1 | 18-05-1998 |
| | | | SK | 97196 A3 | 04-06-1997 |
| | | | WO | 9520621 A1 | 03-08-1995 |
| | | | US | 6013590 A | 11-01-2000 |
| | | | ZA | 9500656 A | 29-05-1995 |
| WO 02077080 | A | 03-10-2002 | AU | 2002254207 B2 | 16-02-2006 |
| | | | BR | 0208377 A | 15-06-2004 |
| | | | CA | 2440544 A1 | 03-10-2002 |
| | | | CN | 1500114 A | 26-05-2004 |
| | | | EP | 1373378 A1 | 02-01-2004 |
| | | | JP | 2004536897 T | 09-12-2004 |
| | | | MX | PA03008886 A | 08-12-2003 |
| WO 9923146 | A | 14-05-1999 | AU | 1281499 A | 24-05-1999 |
| | | | EP | 1025152 A1 | 09-08-2000 |
| US 5618855 | A | 08-04-1997 | NONE | | |
| WO 9929220 | A | 17-06-1999 | DE | 69816653 D1 | 28-08-2003 |
| | | | DE | 69816653 T2 | 13-05-2004 |
| | | | EP | 1035793 A1 | 20-09-2000 |
| | | | JP | 3055778 B2 | 26-06-2000 |
| | | | JP | 11239517 A | 07-09-1999 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 08 25 0822

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2008

| Patent document cited in search report | | Publication date | Patent family member(s) | | Publication date |
|---|---|---|---|---|---|
| WO 9929220 | A | | TW | 501928 B | 11-09-2002 |
| CN 1313348 | A | 19-09-2001 | NONE | | |
| CN 1405220 | A | 26-03-2003 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000139755 A
- JP 2004248910 A